# EUROPEAN PATENT APPLICATION

(11) **EP 2 037 278 A1**
(43) Date of publication of application: **18.03.2009**
(21) Application number: 07116151.7
(22) Date of filing: 11.09.2007
(51) Int. Cl.: G01N 33/96

(54) **Vascular markers in the remodelling of cardiac injury**

(71) Applicant: F.HOFFMANN-LA ROCHE AG, 4070 Basel (CH); Roche Diagnostics GmbH, 68305 Mannheim (DE)
(72) Inventor: The designation of the inventor has not yet been filed
(74) Representative: Dick, Alexander

(57) **Abstract**

The present invention is concerned with diagnostic means and methods. More specifically, the present invention relates to a method for diagnosing the angiogenic status of a subject suffering from myocardial infarction comprising determining the amounts of PLGF, sFLT1 and Endoglin in a first sample of a subject obtained after myocardial infarction and in a second sample of the said subject obtained after the said first sample and comparing the said amounts in the first sample with those in the second sample whereby the angiogenic status is diagnosed. The present invention also encompasses a method of determining whether a subject suffering from myocardial infarction is susceptible to a pro-angiogenic therapy. Finally, the present invention relates to a kit or a device for carrying out the method of the invention.

## Description

The present invention is concerned with diagnostic means and methods. More specifically, the present invention relates to a method for diagnosing the angiogenic status of a subject suffering from myocardial infarction comprising determining the amounts of PLGF, sFLT1 and Endoglin in a first sample of a subject obtained after myocardial infarction and in a second sample of the said subject obtained after the said first sample and comparing the said amounts in the first sample with those in the second sample whereby the angiogenic status is diagnosed. The present invention also encompasses a method of determining whether a subject suffering from myocardial infarction is susceptible to a pro-angiogenic therapy. Finally, the present invention relates to a kit or a device for carrying out the method of the invention.

Myocardial infarction (MI) is a life threatening acute cardiovascular event. It is caused by an impaired oxygen support of the myocardium resulting from occlusion, stenosis of coronary blood vessels or an otherwise insufficient blood flow within the coronary vessel system. Occlusion or stenosis of the coronary blood vessels may be the result of, e.g., atherosclerotic changes of the blood vessels or other thrombotic events. MI affects the function of the heart and, in particular, its electrophysiology resulting in ventricular fibrillation or tachycardia.

MI is usually accompanied by ischemia of parts of the myocardium followed by cardiac necrosis which can be monitored by the release of cardiac troponins, e.g. Troponin I or T, into the blood. As a result of the cardiac necrosis, a vascular remodelling process takes place in the affected areas which includes angiogenesis. Angiogenesis is known as the formation of new blood vessels from already existing blood vessels by a capillary sprouting process. The process is under physiological conditions driven by angiogenic growth factors such as the vascular endothelial growth factor (VEGF). The expression of such angiogenic growth factors is regulated pivotally by hypoxia. Thus, if a tissue becomes ischemic, the cells will start to produce angiogenic growth factors which will attract new blood vessels to the affected tissue by angiogenesis.

Also after MI angiogenesis plays a crucial role in the restoration of the myocardium. However, the capability of a subject for angiogenesis, i.e. its angiogenic status, is dependent on complex biological parameters. Various angiogenesis promoting factors as well as inhibitors of angiogenesis have been reported (Nyberg 2005, Cancer Res 65:3967-3979).

As set forth above already, various factors besides VEGF have been reported to play a role in angiogenesis. Placental growth factor (P1GF) is a closely related growth factor suggested to play a role in the related process of arteriogenesis together with its putative receptor Flt-1 (Khurana 2005, Circulation 111:2828-2836). Other factors which are possibly involved in arteriogenesis and angiogenesis are the members of the Transforming growth factor-beta superfamily as well as their receptors or binding partners such as the ALK receptors or Endoglin (van Laake 2006, Circulation, 114:2288-2297; Bobik 2006, Arterioscler Thromb Vasc Biol 26: 1712-1720; Bertolino 2005, Chest Supplement 128: 585-590). Fibroblast growth factor (FGF), Platlet derived growth factor (PDGF) as well as cytokines and matrix-metalloproteinases have been also described as potent angiogenic factors (Nyberg, loc.cit.).

It is to be understood from the above that it is highly desirable to determine the angiogenic status of a subject suffering from MI. Based on such an assessment of the angiogenic status, it can be predicted whether a subject will be susceptible for a pro-angiogenic therapy in order to improve its long-term perspectives.

Thus, the technical problem underlying the present invention could be seen as the provision of means and methods for determining the angiogenic status of a subject after MI. Thereby, a suitable therapy can be selected in order to improve a subjects long-term perspective. The technical problem is solved by the embodiments characterized in the accompanying claims and herein below.

Accordingly, the present invention relates to a method for diagnosing the angiogenic status of a subject suffering from myocardial infarction comprising:
a) determining the amounts of PLGF, sFLT1 and Endoglin in a first sample of a subject obtained after myocardial infarction;
b) determining the amounts of PLGF, sFLT1 and Endoglin in a second sample of the said subject obtained after the said first sample; and
c) comparing the amounts determined in step a) with the amounts determined in step b), whereby the angiogenic status is diagnosed.

The method of the present invention, preferably, is an in vitro method. Moreover, it may comprise steps in addition to those explicitly mentioned above. For example, further steps may relate to sample pre-treatments or evaluation of the results obtained by the method. The method of the present invention may be also used for monitoring, confirmation, and subclassification of the angiogenic status. The method may be carried out manually or assisted by automation. Preferably, step (a), (b) and/or (c) may in total or in part be assisted by automation, e.g., by a suitable robotic and sensory equipment for the determination in step (a) and (b) or a computer-implemented comparison in step (c).

The term "diagnosing" as used herein refers to assessing the probability according to which a subject has a certain angiogenic status, i.e. a pro-angiogenic or an anti-angiogenic status, referred to in this specification. As will be understood by those skilled in the art, such an assessment is usually not intended to be correct for 100% of the subjects to be diagnosed. The term, however, requires that a statistically significant portion of subjects can be correctly diagnosed to exhibit the said angiogenic status. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population.

The term "angiogenic status" as used herein refers to the capability of a subject of forming blood vessels from already existing blood vessels, e.g., by sprouting. Specifically, it has been found that depending on the changes of the amounts of the molecules referred to herein present in a subject after MI, angiogenesis may occur without further ado, i.e. by the physiological initiators and, preferably, by hypoxia, or may further require exogenously supplied initiators such as angiogenic drugs specified elsewhere in the description. Thus, the angiogentic status, i.e. the capability for angiogenesis in a subject, is determined by the physiological constitution of a subject with respect to the aforementioned molecules.

The term "subject" as used herein relates to animals, preferably mammals, and, more preferably, humans. However, it is envisaged by the present invention that the subject shall be suffering from coronary heart disease as specified elsewhere herein.

The term "myocardial infarction (MI)" refers to an acute cardiovascular event caused by an impaired oxygen supply of the myocardium. As a result of the impaired oxygen supply, ischemia and, subsequently, necrosis occurs in the affected areas of the myocardium. Due to the said cardiac necrosis, cardiac troponins, preferably Troponin I and/or T, will be released from the affected cells of the myocardium into the blood. Preferably, MI affects the physiological function of the heart and, in particular, its electrophysiology resulting in ventricular fibrillation or tachycardia. Further symptoms are chest pain (typically extending into the left arm) shortness of breath, nausea, vomiting, palpitations, sweating, and anxiety. Women often experience different symptoms from men. The most common symptoms of MI in women include shortness of breath, weakness, and fatigue. Approximately one third of all myocardial infarctions are, however, silent, without any of the aforementioned symptoms. Preferably, MI results from occlusion or stenosis of coronary blood vessels or an otherwise insufficient blood flow within the coronary vessel system. Occlusion or stenosis of the coronary blood vessels may be the result of, e.g., atherosclerotic changes of the blood vessels, other thrombotic events in connection with coronary heart diseases.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein. A "first sample" as used herein refers to a sample which has been obtained from the subject immediately after the MI has occurred or become apparent by the characteristic symptoms. A first sample can be immediately obtained, preferably, within the first five hours after MI, more preferably up to three hours after MI. The "second sample" according to the invention shall have been obtained after the first sample. The second sample is, preferably, obtained after the remodelling processes have started. More preferably, the second sample is obtained between one and four months and, most preferably, three month after the first sample has been obtained or after MI occurred.

The term "P1GF (Placental Growth Factor)" as used herein refers to a placenta derived growth factor which is a 149-amino-acid-long polypeptide and is highly homologous (53% identity) to the platelet-derived growth factor-like region of human vascular endothelial growth factor (VEGF). Like VEGF, P1GF has angiogenic activity in vitro and in vivo. For example, biochemical and functional characterization of P1GF derived from transfected COS-1 cells revealed that it is a glycosylated dimeric secreted protein able to stimulate endothelial cell growth in vitro (Maqlione1993, Oncogene 8(4):925-31). Preferably, P1GF refers to human P1GF, more preferably, to human P1GF having an amino acid sequence as shown in Genebank accession number P49763, GI: 17380553 (Genebank is available from the NCBI, USA under www.ncbi.nlm.nih.gov/entrez). Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific P1GF. The degree of identity between two amino acid sequences, in principle, can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants may be allelic variants, splice variants or any other species specific homologs, paralogs, or orthologs. Moreover, the variants referred to herein include fragments of the specific P1GF or the aforementioned types of variants as long as these fragments have the essential immunological and biological properties as referred to above. Such fragments may be, e.g., degradation products of P1GF. Further included are variants which differ due to posttranslational modifications such as glycosylation, phosphorylation or myristylation.

The term "Endoglin" as used herein refers to a polypeptide having a molecular weight of 180 kDa non-reduced, 95 kDa after reduction and 66 kDa in its reduced and N-deglycosylated form. The polypeptide is capable of forming dimmers and bins to TGF-β and TGF-β receptors (see below). Endoglin may be phosphorylated. Preferably, Endoglin refers to human Endoglin. More preferably, human Endoglin has an amino acid sequence as shown in Genebank accession number AAC63386.1, GI: 3201489. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific Endoglin. Variants may be allelic variants, splice varaiants or any other species specific homologs, paralogs, or orthologs. Moreover, the variants referred to herein include fragments of the specific Endoglin or the aforementioned types of variants as long as these fragments have the essential immunological and biological properties as referred to above. Such fragments may be, e.g., degradation products of Endoglin. Further included are variants which differ due to posttranslational modifications such as glycosylation, phosphorylation or myristylation.

The term "soluble (s)Flt-1" as used herein refers to polypeptide which is a soluble form of the VEGF receptor FLT1. It was identified in conditioned culture medium of human umbilical vein endothelial cells. The endogenous soluble FLT1 (sFLT1) receptor is chromatographically and immunologically similar to recombinant human sFLT1 and binds [1251] VEGF with a comparable high affinity. Human sFLT1 is shown to form a VEGF-stabilized complex with the extracellular domain of KDR/Flk-1 in vitro. Preferably, sFLT1 refers to human sFLT1. More preferably, human sFLT1 can be deduced from the amino acid sequence of Flt-1 as shown in Genebank accession number P17948, GI: 125361. An amino acid sequence for mouse sFLT1 is shown in Genebank accession number BAA24499.1, GI: 2809071. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFLT1. Variants may be allelic variants, splice variants or any other species specific homologs, paralogs, or orthologs. Moreover, the variants referred to herein include fragments of the specific sFLT1 or the aforementioned types of variants as long as these fragments have the essential immunological and biological properties as referred to above. Such fragments may be, e.g., degradation products of sFLT1. Further included are variants which differ due to posttranslational modifications such as glycosylation, phosphorylation or myristylation.

Determining the amount of the polypeptides referred to in this specification relates to measuring the amount or concentration, preferably semi-quantitatively or quantitatively. Measuring can be done directly or indirectly. Direct measuring relates to measuring the amount or concentration of the polypeptide based on a signal which is obtained from the polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse-proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a polypeptide comprises the steps of (a) contacting a cell capable of eliciting a cellular response the intensity of which is indicative of the amount of the polypeptide with the said polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a polypeptide comprises the step of measuring a specific intensity signal obtainable from the polypeptide in the sample. As described above, such a signal may be the signal intensity observed at a mass to charge (m/z) variable specific for the polypeptide observed in mass spectra or a NMR spectrum specific for the polypeptide.

Determining the amount of a polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/polypeptide" complex or the ligand which was bound by the polypeptide, respectively, may be contacted with a suitable substrate allowing detection by the generation of an intensity signal. For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a coloured reaction product, fluorescence or chemo luminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a polypeptide may be, also preferably, determined as follows: (a) contacting a solid support comprising a ligand for the polypeptide as specified above with a sample comprising the polypeptide and (b) measuring the amount polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide, the relative amount or concentration of the said polypeptide as well as any value or parameter which correlates thereto or can be derived therefrom. Such values or parameters comprise intensity signal values from all specific physical or chemical properties obtained from the said polypeptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the polypeptides or intensity signals obtained from specifically bound ligands. It is to be understood that values correlating to the aforementioned amounts or parameters can also be obtained by all standard mathematical operations.

The term "comparing" as used herein encompasses comparing the amount of each of the polypeptides comprised by the first sample to be analyzed with the corresponding amounts of each of the polypeptides in the second sample. In other words, the amounts of PLGF in the first and in the second sample are compared to each other and the same comparison is carried out mutatis mutandis for the amounts of sFLT1 and Endoglin. It is to be understood that comparing as used herein refers to a comparison of corresponding parameters or values, e.g., an absolute amount is compared to an absolute amount while a concentration is compared to a concentration or an intensity signal obtained in a first sample is compared to the same type of intensity signal of a second sample. The comparison referred to in step (c) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format.

In principle, it has been found that a pro-angiogenic status in a subject after MI is accompanied by a decrease of the biomarkers PLGF and sFLT1 while the biomarker Endoglin will increase. Theses changes in combination are not for a subject showing an anti-angiogenic status after MI, i.e. all cases in which the subjects do show a pro-angiogenic status.

Thus, it will be understood from the forgoing that in a preferred embodiment of the method of the present invention, a decreased amount of PLGF and sFLT1 and an increased amount of Endoglin in the second sample with respect to the first sample is indicative for a pro-angiogenic status. In cases where either the Endoglin is not increasing or PLGF and/or sFLT1 are not decreasing, an anti-angiogenic status is to be diagnosed.

Advantageously, it has been found in the study underlying the present invention that a combination of P1GF, Endoglin and sFLT1 as biomarkers are required to determine the angiogenic status of a subject after MI in a reliable and efficient manner. Moreover, it has been found that each of said biomarkers is statistically independent from each other. Accordingly, the method of the present invention provides for a highly reliable diagnosis. As described above, the techniques which are currently used to resolve this issue are time consuming and cost intensive. The method of the present invention, however, allows a reliable, fast and less cost intensive diagnosis and can be implemented even in portable assays, such as test stripes. Therefore, the method is particularly well suited for diagnosing emergency patients. Thanks to the findings of the present invention, a suitable angiogenic therapy for a subject suffering from MI and its consequences can be reliably selected. Severe side effects caused by the wrong treatment of patients can be avoided.

The present invention, furthermore, relates to a method of determining whether a subject suffering from myocardial infarction is susceptible to a pro-angiogenic therapy comprising:
a) determining the amounts of PLGF, sFLT1 and Endoglin in a first sample of a subject obtained after myocardial infarction;
b) determining the amounts of PLGF, sFLT1 and Endoglin in a second sample of the said subject obtained after the said first sample; and
b) comparing the amounts determined in step a) with the amounts determined in step b), whereby it is determined whether the subject is susceptible to a pro-angiogenic therapy.

The term "pro-angiogenic therapy" as recited above relates to a therapy which induces or enhances the process of angiogenesis systemically or topically in a subject. Preferably, said pro-angiogenic therapy comprises administration of an pro-angiogenic drug, preferably, selected from the group consisting of: VEGF, P1GF, Endoglin, anti-Flt-1 antibodies and ALK5 modifiers.

The term "susceptible" as used herein means that a statistically significant portion of subjects identified by the method as being susceptible respond to the envisaged therapy by showing angiogenesis in the affected areas of the heart.

In a preferred embodiment of the aforementioned method, a decreased amount of PLGF and sFLT1 and a increased amount of Endoglin in the second sample with respect to the first sample exclude a subject as being susceptible to a pro-angiogenic therapy.

The present invention also relates to a device for diagnosing the angiogenic status of a subject suffering from myocardial infarction comprising:
a) means for determining the amounts of PLGF, sFLT1 and Endoglin in a first and second sample of a subject wherein said first sample has been obtained after myocardial infarction and said second sample has been obtained after said first sample; and
b) means for comparing the amounts of PLGF, sFlT1 and Endoglin determined by the means of a) in the first sample with the corresponding amounts determined in the second sample, whereby the diagnosis of the angiogenic status is allowed.

The term "device" as used herein relates to a system of means comprising at least the aforementioned means operatively linked to each other as to allow the prediction. Preferred means for determining the amount of the said polypeptides and means for carrying out the comparison are disclosed above in connection with the method of the invention. How to link the means in an operating manner will depend on the type of means included into the device. For example, where means for automatically determining the amount of the polypeptides are applied, the data obtained by said automatically operating means can be processed by, e.g., a computer program in order to diagnose the angiogenic status. Preferably, the means are comprised by a single device in such a case. Said device may accordingly include an analyzing unit for the measurement of the amount of the polypeptides in a sample and a computer unit for processing the resulting data for the differential diagnosis. Alternatively, where means such as test stripes are used for determining the amount of the polypeptides, the means for diagnosing may comprise accompanied with a pro- or anti-angiogenic status. The test stripes are, preferably, coupled to a ligand which specifically binds to the polypeptides as defined elsewhere in this specification. The strip or device, preferably, comprises means for detection of the binding of said peptides to the said ligand. Preferred means for detection are disclosed in connection with embodiments relating to the method of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician. Further preferred devices comprise the analyzing units/devices (e.g., biosensors, arrays, solid supports coupled to ligands specifically recognizing the polypeptides, Plasmon surface resonace devices, NMR spectrometers, mass- spectrometers etc.) or evaluation units/devices referred to above in accordance with the method of the invention.

Finally, the present invention encompasses a kit adapted for carrying out the method of the present invention comprising:
a) means for determining the amounts of PLGF, sFLT1 and Endoglin in a first and second sample of a subject wherein said first sample has been obtained after myocardial infarction and said second sample has been obtained after said first sample; and
b) means for comparing the amounts of PLGF, sF1T1 and Endoglin determined by the means of a) in the first sample with the corresponding amounts determined in the second sample, whereby the diagnosis of the angiogenic status is allowed.

The term "kit" as used herein refers to a collection of the aforementioned means, preferably, provided in separately or within a single container. The container, also preferably, comprises instructions for carrying out the method of the present invention. The invention, thus, relates to a kit comprising a means or an agent for measuring a polypeptide referred to herein. Examples for such means or agents as well as methods for their use have been given in this specification. The kit, preferably, contains the aforementioned means or agents in a ready-to-use manner. Preferably, the kit may additionally comprise instructions, e.g., a user's manual for interpreting the results of any determination(s) with respect to the diagnoses provided by the methods of the present invention. Particularly, such manual may include information for allocating the amounts of the determined polypeptides to the kind of diagnosis. Details are to be found elsewhere in this specification. Additionally, such user's manual may provide instructions about correctly using the components of the kit for determining the amount(s) of the respective biomarker. A users manual may be provided in paper or electronic form, e.g., stored on CD or CD ROM. The present invention also relates to the use of said kit in any of the methods according to the present invention.

All references cited in this specification are herewith incorporated by reference with respect to their entire disclosure content and the disclosure content specifically mentioned in this specification.

The figure show:
**Figure 1****:** A box plot analysis is shown for the amounts of PLGF, sFLT1 and Endoglin as determined at time point = 0, i.e. three days after MI, and at time point = 3 months. 5^{th}, 25^{th}, 75^{th}, and 95^{th} percentiles (perc.) as well as the median values are indicated in the table. Moreover, the relative changes of the amounts have been calculated (% changes).
**Figure 2****:** A linear regression analysis is shown for sFLT1 and PLGF demonstrating that both biomarkers are statistically independent from each other at time point=0.
**Figure 3****:** A linear regression analysis is shown for sFLT1 and PLGF demonstrating that both biomarkers are statistically independent from each other at time point=3 months.
**Figure 4****:** A linear regression analysis is shown for Endoglin and PLGF demonstrating that both biomarkers are statistically independent from each other at time point=0.
**Figure 5****:** A linear regression analysis is shown for Endoglin and PLGF demonstrating that both biomarkers are statistically independent from each other at time point=3 months.
**Figure 6****:** A linear regression analysis is shown for sFLT1 and Endoglin demonstrating that both biomarkers are statistically independent from each other at time point=0.
**Figure 7****:** A linear regression analysis is shown for sFLT1 and Endoglin demonstrating that both biomarkers are statistically independent from each other at time point=3 months.

The following Example merely illustrates the invention. It shall, whatsoever, not be construed as a limitation of the scope of the invention.

**Example:** P1GF, sFLT1 and Endoglin are statistically independent common predictors for the angiogenic status in patients suffering from myocardial infarction

A total of 140 patients suffering from myocardial infarction were investigated for blood levels of sFLT1, Endoglin, P1GF and TGF-β1. All patients showed apparently a pro-angiogenis status as confirmed by echocardiography testing of heart physiology approx. three month after myocardial infarction.

Blood levels of sFLT1, P1GF and Endoglin were determined at a first time point (three days after myocardial infarction took place) and a second time point (three month after the first time point) using the commercially available Immunoassays "Quantikine" (Catalog numbers DVR100B, DPG00 and DNDG00) from R & D Systems, USA.

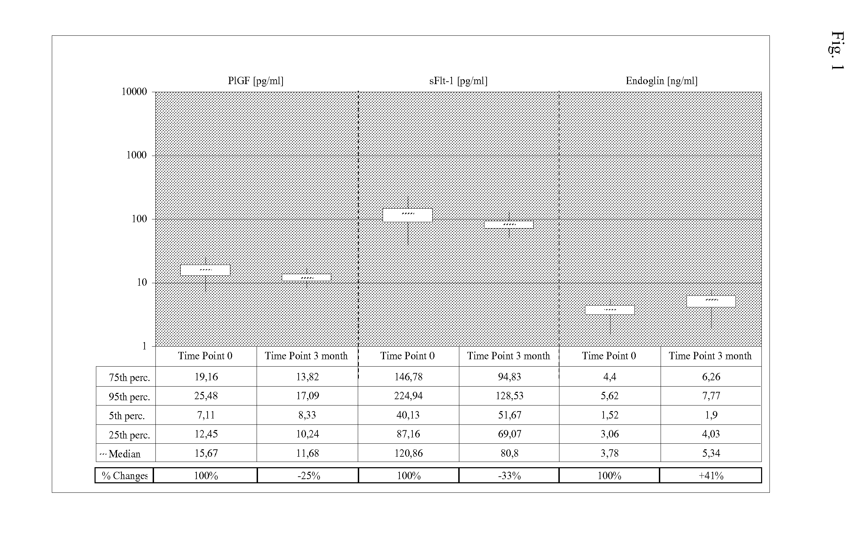

The results of the study are summarized in Figure 1. Specifically, a decrease of PLGF and sFLT1 levels was observed in the pro-angiogenic patients accompanied by an increase of the Endoglin level.

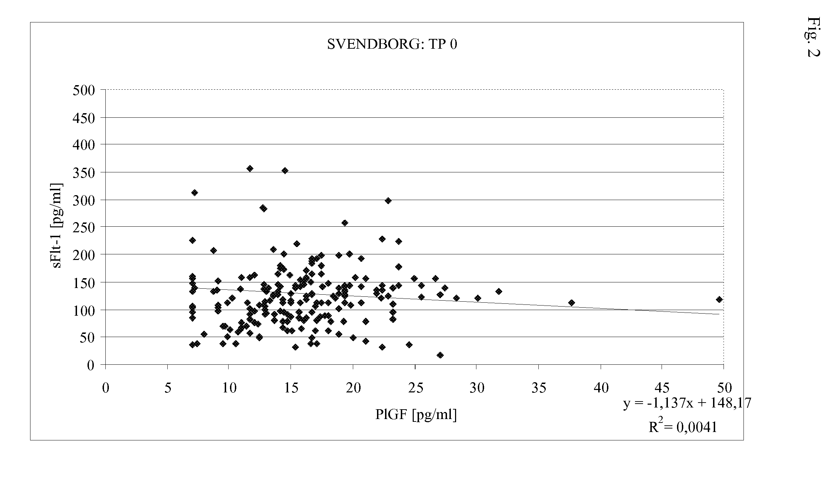

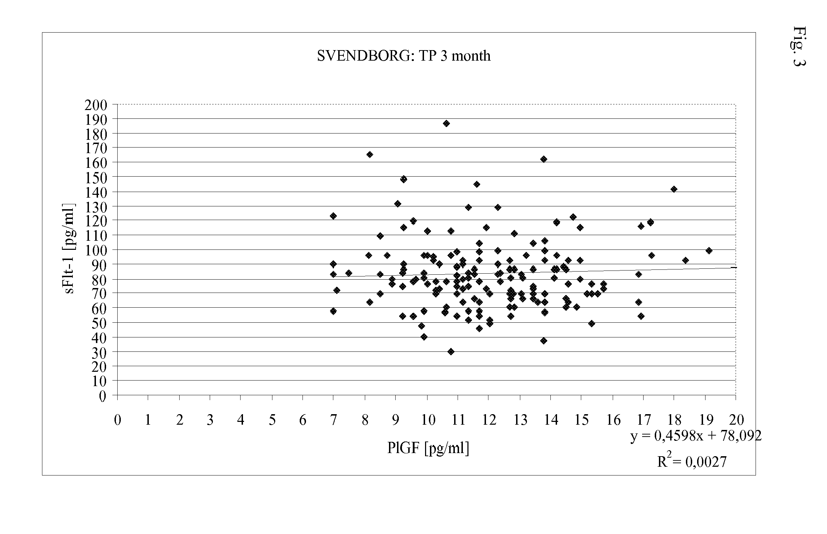

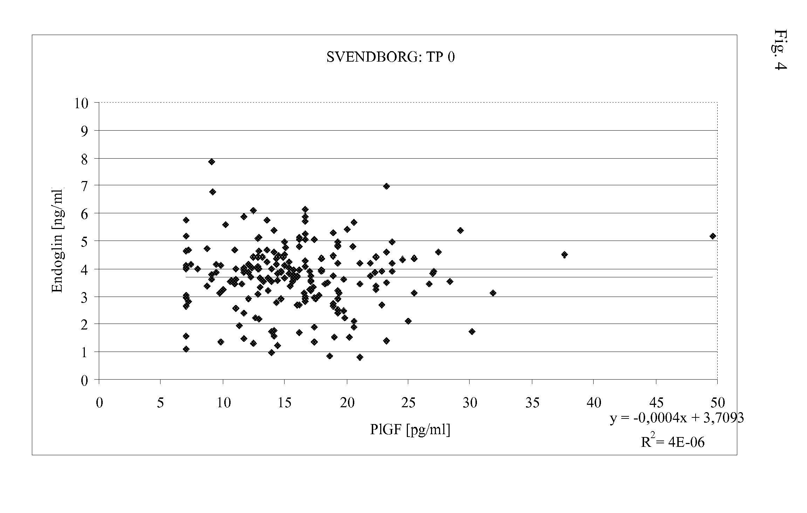

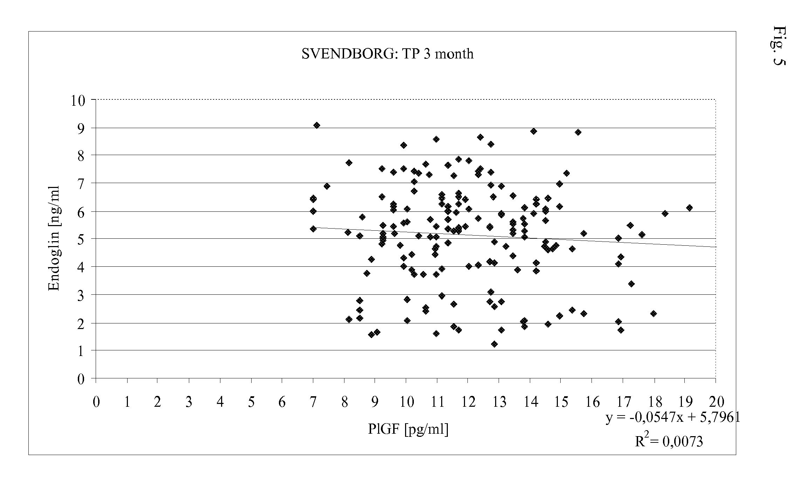

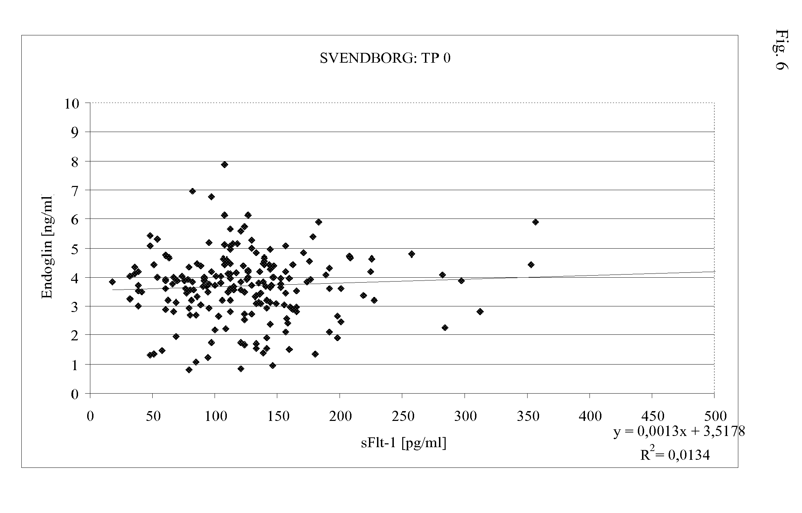

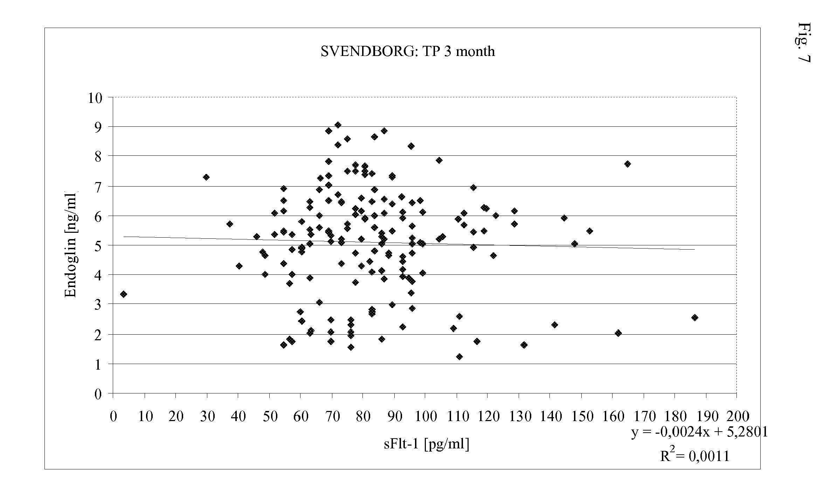

Moreover, P1GF, Endoglin and sFLT1 were statistically independent from each other as shown by linear regression analysis (Figure 2 to 7) at time point=0 as well as time point=3 months.

## Claims

1. A method for diagnosing the angiogenic status of a subject suffering from myocardial infarction comprising:
a) determining the amounts of PLGF, sFLT1 and Endoglin in a first sample of a subject obtained after myocardial infarction;
b) determining the amounts of PLGF, sFLT1 and Endoglin in a second sample of the said subject obtained after the said first sample; and
b) comparing the amounts determined in step a) with the amounts determined in step b), whereby the angiogenic status is diagnosed.

2. The method of claim 1, wherein a decreased amount of PLGF and sFLT1 and an increased amount of Endoglin in the second sample with respect to the first sample is indicative for an pro-angiogenic status.

3. A method of determining whether a subject suffering from myocardial infarction is susceptible to a pro-angiogenic therapy comprising:
a) determining the amounts of PLGF, sFLT1 and Endoglin in a first sample of a subject obtained after myocardial infarction;
b) determining the amounts of PLGF, sFLT1 and Endoglin in a second sample of the said subject obtained after the said first sample; and
b) comparing the amounts determined in step a) with the amounts determined in step b), whereby it is determined whether the subject is susceptible to a pro-angiogenic therapy.

4. The method of claim 3, wherein a decreased amount of PLGF and sFLT1 and an in creased amount of Endoglin in the second sample with respect to the first sample exclude a subject as being susceptible to a pro-angiogenic therapy.

5. The method of claim 3 or 4, wherein said pro-angiogenic therapy comprises administration of a pro-angiogenic drug.

6. The method of any one of claims 1 to 5, wherein said first sample has been obtained within 3 days after myocardial infarction.

7. The method of any one of claims 1 to 6, wherein said second sample has been obtained more than 3 days and within 3 months after myocardial infarction.

8. The method of any one of claims 1 to 7, wherein said subject is a human.

9. A device for diagnosing the angiogenic status of a subject suffering from myocardial infarction comprising:
a) means for determining the amounts of PLGF, sFLT1 and Endoglin in a first and second sample of a subject wherein said first sample has been obtained after myocardial infarction and said second sample has been obtained after said first sample; and
b) means for comparing the amounts of PLGF, sF1T1 and Endoglin determined by the means of a) in the first sample with the corresponding amounts determined in the second sample, whereby the diagnosis of the angiogenic status is allowed.

10. A kit adapted for carrying out the method of any one of claims 1 to 8 comprising:
a) means for determining the amounts of PLGF, sFLT1 and Endoglin in a first and second sample of a subject wherein said first sample has been obtained after myocardial infarction and said second sample has been obtained after said first sample; and
b) means for comparing the amounts of PLGF, sF1T1 and Endoglin determined by the means of a) in the first sample with the corresponding amounts determined in the second sample, whereby the diagnosis of the angiogenic status is allowed.
